# EUROPEAN PATENT APPLICATION

(11) **EP 1 894 582 A1**
(43) Date of publication of application: **05.03.2008**
(21) Application number: 06745959.4
(22) Date of filing: 01.05.2006
(51) Int. Cl.: A61L 31/00, A61K 31/506, A61K 47/32, A61P 9/02, A61P 9/12

(54) **METHOD FOR CONTROL OF DRUG ELUTION RATE AND COMPOSITION FOR COATING OF DRUG-ELUTING STENT**

(30) Priority: 24.06.2005 JP 2005184350
(71) Applicant: Tokyo Medical and Dental University, Tokyo 113-8510 (JP)
(72) Inventor: AZUMA, H., c/o Tokyo Medical and Dental Uni., Tokyo 113-8510 (JP); NIIMI, Y., c/o Tokyo Medical and Dental Uni., Tokyo 113-8510 (JP); IWASAKI, Y., c/o Tokyo Medical and Dental Uni., Tokyo 113-8510 (JP); NAKABAYASHI, N., c/o Tokyo Medical and Dental Uni., Tokyo 113-8510 (JP)
(74) Representative: Hart Davis, Jason
(86) International application number: PCT/JP2006/309108
(87) International publication number: WO 2006/137222

(57) **Abstract**

Disclosed are a method for controlling the elution of a drug from a stent and a drug-eluting stent capable of reducing the restenosis rate after therapy. A coating composition for a drug-eluting stent comprising an endothelin receptor antagonist and a copolymer of 2-methacrylcyloxyethyl phosphorylcholine and n-butyl methacrylate. The elution of the endothelin receptor antagonist from the coating composition can be controlled by varying the ratio of 2-methacryloyloxyethyl phosphorylcholine to n-butyl methacrylate in the copolymer.

## Description

### TECHNICAL FIELD

The present invention relates to a drug-eluting stent, and particularly a method for control of drug elution rate of a coating composition for a drug-eluting stent containing an antithrombotic polymer and an endothelin receptor antagonist.

### BACKGROUND ART

With respect to the death ratio of Japanese patient with adult disease, the ratio of death caused by cardiac disease has been increased. A blood vessel referred to as a coronary artery around a heart supplies oxygen and nutrition to a heart. Stenocardia is in a condition that arteriosclerosis is developed in the blood vessel, the caliber of the blood vessel narrowed, whereby oxygen and nutrition cannot be supplied sufficiently. Myocardial infarction is in a condition that arteriosclerosis is further developed in the blood vessel, and blood is hardly flow, whereby oxygen and nutrition cannot be supplied practically.

Depending on the kind or severity of such stenocardia and myocardial infarction, a treatment with oral administration, a surgical treatment, and a medical treatment have been applied in order to secure the amount of oxygen when oxygen is delivered insufficient.

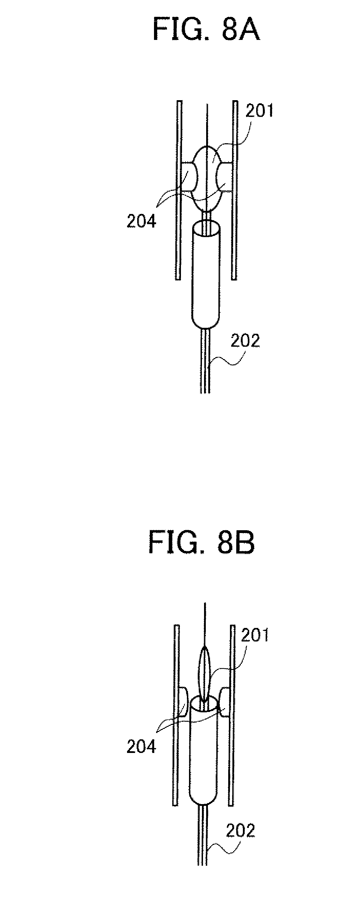

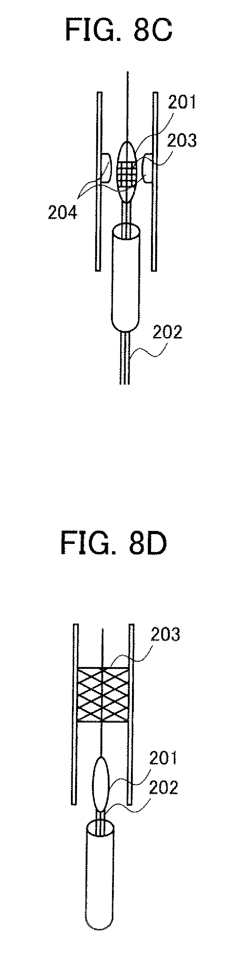

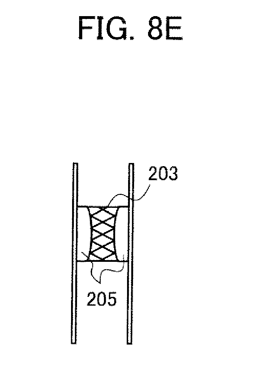

The medical treatment includes a catheter treatment as shown in Fig. 8. The catheter treatment is a method in which a small tube referred to as a catheter is inserted in an artery, and then widens a stenosis or imperforate site. The catheter treatment mainly includes a balloon treatment (balloon therapy) shown in Figs. 8A and 8E, and a stent treatment shown in Figs. 8C and 8D. The balloon treatment is a treatment in which catheter 202 which is a small tube with an elongated balloon 201 hereinafter referred to as a balloon at the tip is passed thorough as shown in Fig. 8A, and this balloon is blown up by applying a pressure at a stenosis or imperforate site 204 to expand the blood vessel, whereby the balloon 201 is removed as shown in Fig. 8B. The balloon treatment has a high success rate and a high safety, and it has also a benefit equivalent to that from a bypass surgery, which is a surgical treatment. However, since a probability to suffer from restenosis is high with 40 to 50%, patients with restenosis are required to repeatedly receive such treatments, resulting that they are extremely stressed.

Thus, the stent 203 shown in Figs. 8C and 8D are generally used for the balloon treatment in order to suppress this restenosis. As shown in Fig. 8C, the stent 203 is a cylinder or coil of metal with a special mesh (stent), which is delivered to the stenosis or imperforate site 204 in an artery, with covering the balloon at the tip of the catheter. As shown in Fig. 8D, the stent 203 is spread out with the balloon 201, and then the stent is placed fixedly, whereby a large lumen is secured, and the expanded site is reinforced. Thus, restenosis hardly occurs. Accordingly, the probability to suffer from restenosis can be reduced to about 20%. Restenosis rate can be reduced greatly by stent placement. However, a vascular wall is damaged by the stent left in the blood vessel, intimal thickening 205 occurs, resulting in a problem such as restenosis as shown in Fig. 8E.

Accordingly, development of the drug-eluting stent in which drug is applied to the surface for suppressing restenosis has been progressed. Restenosis rate can be reduced to less than 10% by using the drug-eluting stent.

As a therapeutic agent for the circulatory failure and the like, 4,5-dihydro-[1H]-benz[g]indazol-3-carboxylic acid derivative or salt thereof having an endothelin receptor antagonism are disclosed in the following Patent Document 1. This compound is useful for prevention, treatment, and the like of diseases involving in endothelin overproduction, such as hypertension, stenocardia, myocardial infarction, cerebral infarction, spasm cerebrovascular, renal insufficiency, hepatic insufficiency, arteriosclerosis, post-PTCA restenosis.

In addition, membrane material for coating biosensors comprising a copolymer of 2-methacryloyloxyethyl phosphorylcholine which is a monomer having a phospholipid polar group and n-butyl methacrylate which is a hydrophobic monomer in the following Patent Document 2. According to this coating material for membrane structure, the sensor enabling to place in a body for a long time can be provided.

Patent Document 1: Unexamined Japanese Patent Publication Application No. H11-29569
Patent Document 2: Japanese Patent No. 2947298

### DISCLOSURE OF THE INVENTION

### Problems to be Solved by the Invention

However, the membrane coating material disclosed in Patent Document 2 is a membrane material for coating biosensors to prevent from deteriorating the material permeability of the coating membrane due to protein adsorption. The membrane coating material has not been studied for the action of other components included in the coating membrane as a membrane coating material for stents.

In addition, since the compound disclosed in Patent Document 1 is administered orally in the form of solid, semisolid, or liquid, the drug concentration of the compound cannot be increased only at a site requiring treatment. If it is possible to efficiently apply the drug at a local site, it will lead to reduce a systemic side effect. In addition, the drug can be eluted easily just by applying the drug to the surface of the stent since the drug is exposed to blood stream when the stent is placed in the blood vessel. Thus, the amount of the drug usable for treatment may be nearly zero at a site requiring an actual treatment. In addition, in order to prolong the effect of the drug over a long term, it is preferable that the drug is eluted over a long term.

The present invention is achieved in view of the above-mentioned problems, and it provides a method for controlling the elution rate of drug in a coating composition and a stent enabling to reduce restenosis rate due to tissue proliferation by coating the coating composition.

### Means for Solving the Problems

As a result of elaborate efforts to achieve the objects, the inventors found that the above-mentioned problems are solved by varying a composition ratio of a copolymer of 2-methacryloyloxyethyl phosphorylcholine and n-butyl methacrylate in the composition so that the elution of an endothelin receptor antagonist effective to stenocardia and myocardial infarction can be controlled. More specifically, the present invention provides the following.

According to a first aspect of the present invention, a method for control of drug elution rate of an endothelin receptor antagonist from a coating composition for a drug-eluting stent comprises: varying a composition ratio of a copolymer of 2-methacryloyloxyethyl phosphorylcholine and n-butyl methacrylate, wherein the coating composition for a drug-eluting stent comprises the endothelin receptor antagonist and the copolymer of 2-methacryloyloxyethyl phosphorylcholine and n-butyl methacrylate.

According to the method for control of drug elution rate of the present invention, the elution of the endothelin receptor antagonist can be easily controlled by varying the composition ratio of the copolymer of 2-methacryloyloxyethyl phosphorylcholine (hereinafter referred to as "MPC") and n-butyl methacrylate (hereinafter referred to as "BMA"), which is hereinafter referred to as "poly (MPC-co-BMA)".

According to a second aspect of the present invention, in the method for control of drug elution rate according to the first aspect of the present invention, the composition ratio by mass of 2-methacryloyloxyethyl phosphorylcholine and n-butyl methacrylate in the copolymer is 0.05:99.95 to 67:33.

According to a third aspect of the present invention, in the method for control of drug elution rate according to the first or second aspect of the present invention, the composition ratio by mass of 2-methacryloyloxyethyl phosphorylcholine and n-butyl methacrylate in the copolymer is 0.5:99.95 to 30:70.

According to this aspect, the composition ratio of MPC and BMA is in the range of 0.05:99.95 to 67:33, and in particular, 0.5:99.5 to 30:70 is preferable, whereby the elution rate of the endothelin receptor antagonist can be controlled. Thus, the desired elution rate can be obtained by varying the composition ratio.

According to a fourth aspect of the present invention, in the method for control of drug elution rate according to any one of the first to three aspects of the present invention, the endothelin receptor antagonist is 4,5-dihydro-[1H]-benz[g]indazol-3-carboxylic acid derivative represented by the following structural formula (1) or salt thereof, 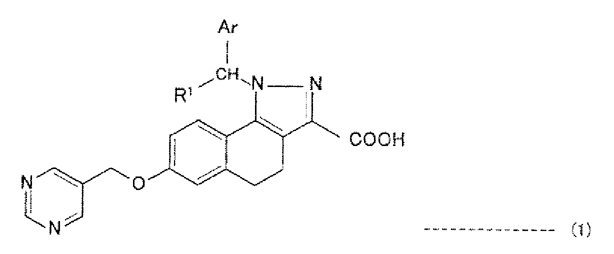 wherein Ar presents a substituted or unsubstituted aryl group; and R¹ represents a hydrogen atom, a substituted or unsubstituted lower alkyl group, a substituted or unsubstituted aryl group, a substituted or unsubstituted cycloalkyl group, or a substituted or unsubstituted heterocyclic group.

According to a fifth aspect of the present invention, in the method for control of drug elution rate according to the fourth aspect of the present invention, 4,5-dihydro-[1H]-benz[g]indazol-3-carboxylic acid derivative or salt thereof is 3-carboxy-4,5-dihydro-1-[1-(3-ethoxyphenyl)propoxy]-7-(5-pyrimidinyl)methoxy-[1H]-benz[g]indazol represented by the following structural formula (2).

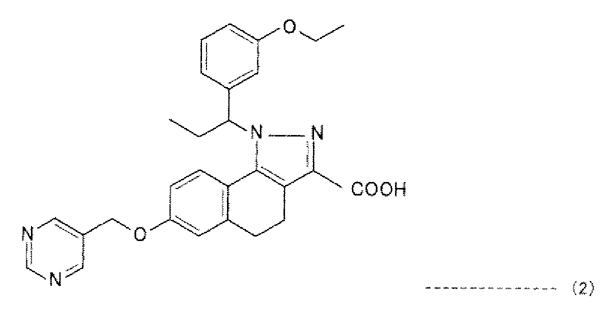

According to a sixth aspect of the present invention, in the method for control of drug elution rate according to any one of the first to three aspects of the present invention, the endothelin receptor antagonist is (1S,2R,3S)-3-[2-(2-hydroxyethoxy)-4-methoxyphenyl]-1-[3,4-(methylenedioxy)phenyl]-5-propoxy-2-indane carboxylic acid (generic name: enrasentan) represented by the following structural formula (3) or [5S-[5α,6β,7α(R*)]]-2-butyl-5-(1,3-benzodioxy-ol-5-yl)-7-[(2-carboxypropyl)-4-methoxyphenyl]-6-dihydro-5H-cyclopenta[b]pyrldine-6-carboxylic acid (cord No.: J-104132) represented by the following structural formula (4).

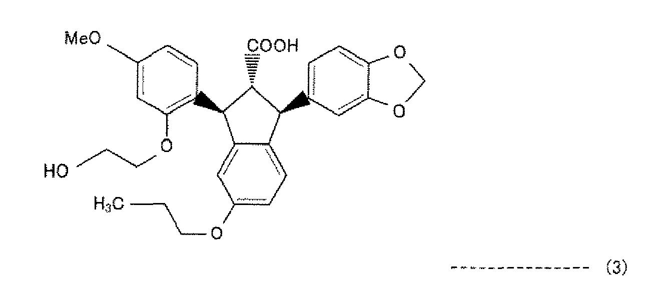

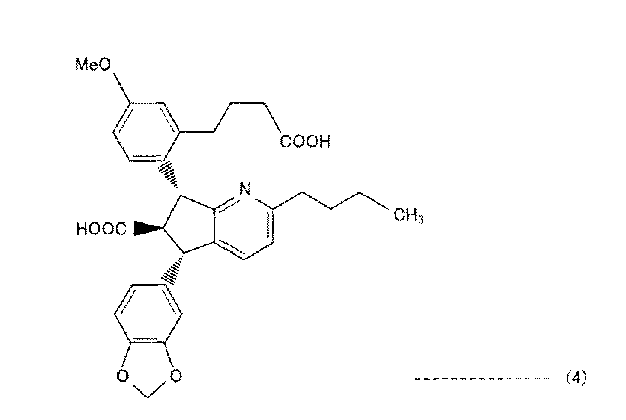

From according to any one of the fourth to sixth aspects of the present invention, the endothelin receptor antagonist is the above-mentioned compounds, whereby the antagonism can exhibit at low concentration.

According to a seventh aspect of the present invention, a composition for coating a drug-eluting stent comprises an endothelin receptor antagonist and a copolymer of 2-methacryloyloxyethyl phosphorylcholine and n-butyl methacrylate.

According to an eighth aspect of the present invention, in the composition for coating a drug-eluting stent according to the seventh aspect of the present invention, the composition ratio by mass of 2-methacryloyloxyethyl phosphorylcholine and n-butyl methacrylate in the copolymer is 0.05:99.95 to 67:33.

According to a ninth aspect of the present invention, in the composition for coating a drug-eluting stent according to the seventh or eighth aspect of the present invention, the composition ratio by mass of 2-methacryloyloxyethyl phosphorylcholine and n-butyl methacrylate in the copolymer is 0.5:99.95 to 30:70.

According to a tenth aspect of the present invention, in the composition for coating a drug-eluting stent according to any one of the seventh to ninth aspects of the present invention, the endothelin receptor antagonist is 4,5-dihydro-[1H]-benz[g]indazol-3-carboxylic acid derivative represented by the following structural formula (1) or salt thereof. 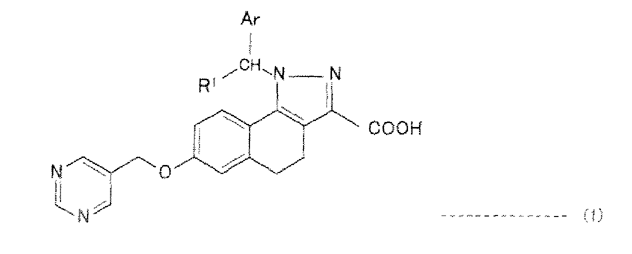 wherein Ar presents a substituted or unsubstituted aryl group; and R¹ represents a hydrogen atom, a substituted or unsubstituted lower alkyl group, a substituted or unsubstituted aryl group, a substituted or unsubstituted cycloalkyl group, or a substituted or unsubstituted heterocyclic group.

According to a eleventh aspect of the present invention, in the composition for coating a drug-eluting stent according to the tenth aspect of the present invention, 4,5-dihydro-[1H]-benz[g]indazol-3-carboxylic acid derivative or salt thereof is 3-carboxy-4,5-dihydro-1-[1-(3-ethoxyphenyl)propoxy]-7-(5-pyrimidinyl)methoxy-[1H]-benz[g]indazol represented by the following structural formula (2).

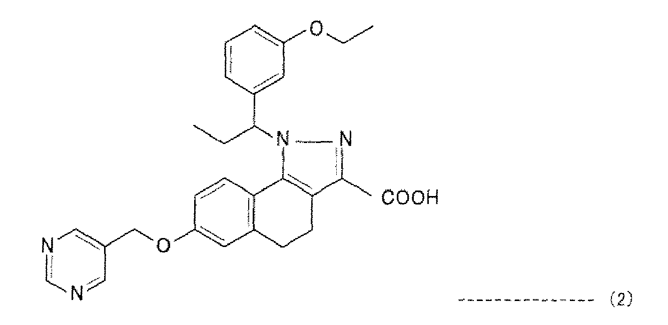

According to a twelfth aspect of the present invention, in the composition for coating a drug-eluting stent according to any one of the seventh to ninth aspects of the present invention, the endothelin receptor antagonist is (1S,2R,3S)-3-[2-(2-hydroxyethoxy)-4-methoxyphenyl]-1-[3,4-(methylenedioxy)phenyl]-5-propoxy-2-indane carboxylic acid represented by the following structural formula (3) or [5S-[5α,6β,7α(R*)]]-2-butyl-5-(1,3-benzodioxy-ol-5-yl)-7-[(2-carboxypropyl)-4-methoxyphenyl]-6-dihydro-5H-cyclopenta[b]pyridine-6-carboxylic acid represented by the following structural formula (4).

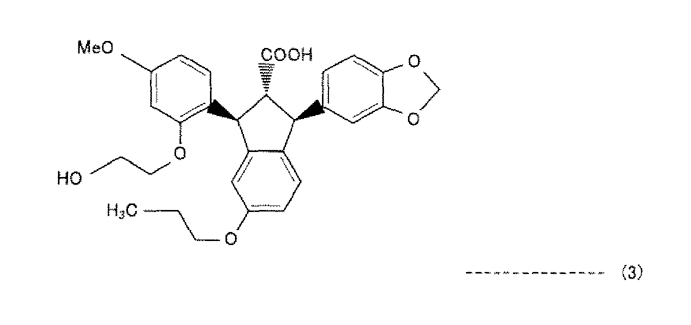

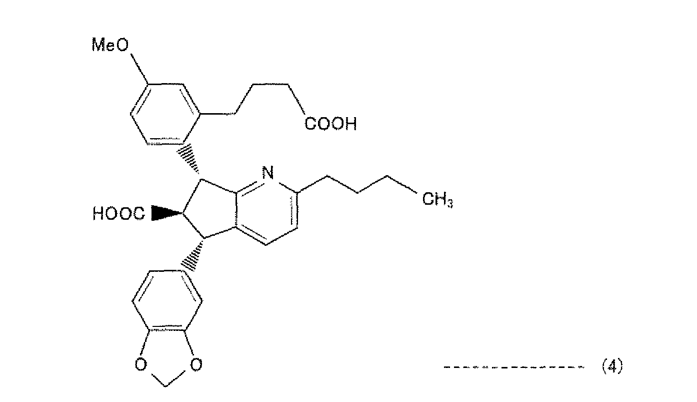

The drug-eluting stent coating composition according to any one of the seventh to twelfth aspects of the present invention is a coating composition to which the method for control of drug elution rate according to any one of the first to sixth aspects of the present invention applies. According to this drug-eluting stent coating composition, the same effect as that obtained from the method for control of drug elution rate as described above can be achieved.

According to a thirteenth aspect of the present invention, a drug-eluting stent is formed by coating the composition for coating a drug-eluting stent according to any one of the seventh to twelfth aspects of the present invention thereon.

The composition for coating a drug-eluting stent according to any one of the seventh to twelfth aspects of the present invention can be used suitably for a stent. In addition, since the elution of the drug is controlled, effect is prolonged over a long term after stent placement.

According to a fourteenth aspect of the present invention, in the drug-eluting stent according to the thirteenth aspect of the present invention, the coating comprises a plurality of coating layers, and the ratio of n-butyl methacrylate in the copolymer of 2-methacryloyloxyethyl phosphorylcholine and n-butyl methacrylate decreases sequentially from the inner layer to the outer layer.

According to this aspect, the elution rate of the drug can freely vary from the outer layer to the inner layer in the plurality of coating layers coating a stent. Thus, the drug can be eluted over a long term, whereby the effect can be prolonged. Particularly, it is preferable that the ratio of n-butyl methacrylate is decreased sequentially from the outer layer to the inner layer. According to this aspect, the drug of the outer layer can be eluted quickly, and the drug of the inner layer can be eluted slowly, whereby the drug can be eluted over a long term.

### Effects of the Invention

According to the present invention, a method for control of drug elution rate in which the elution rate of the drug can be controlled according to the object can be provided. In addition, a composition for coating a drug-eluting stent (hereinafter referred to as "composition") enabling to prevent or reduce postangioplasty restenosis and a drug-eluting stent therewith can be provided over a long term.

### BRIEF DESCRIPTION OF THE DRAWINGS

Fig. 1 is a pattern diagram illustrating a verification test of stenosis development.
Fig. 2 is enlarged section photographs of rabbit common carotid artery at pretest and posttest.
Fig. 3 is a graph showing variation with time of the cumulative amount of the compound represented by the structural formula (2).
Fig. 4 is a schematic diagram of stent placement described in Preparation 1.
Fig. 5 is a tissue image around a metal stent at posttest of the stent in Preparation 1.
Fig. 6 is a tissue image around a metal stent at posttest of the stent in Preparation 2.
Fig. 7 is a tissue image around a metal stent at posttest of the stent in Preparation 3.
Fig. 8 is a figure illustrating the medical treatment.

### PREFERRED MODE FOR CARRYING OUT THE INVENTION

### Composition

The composition for drug-eluting stent according to the present invention contains poly (MPC-co-BMA) and an endothelin receptor antagonist. Each component is explained as follows. Poly (MPC-co-BMA)

Poly (MPC-co-BMA) used in the present invention can be synthesized by way of each of methods described in Japanese Patent No. 2947298 and Polymer Journal 22,355 (1990). Poly (MPC-co-BMA) can be synthesized by using MPC and BMA at the mole ratio of preferably from 2:98 to 50:50, more preferably from 5:95 to 40:60, and reacting thereof in a mixed solvent, preferably tetrahydrofuran (hereinafter referred to as "THF") and ethanol at 60 to 65°C for 4 to 20 hours, in the presence of an initiator, preferably α,α'-azobisisobutyronitrile (AIBN).

The elution of the endothelin receptor antagonist can be controlled by varying the composition ratio (mass ratio) of MPC and BMA in the copolymer of MPC and BMA. The elution can be accelerated by reducing the ratio of BMA, which is a hydrophobic monomer, and it can be retarded by increasing ratios of BMA to retard the elution, whereby the elution can be controlled. The composition ratio (mass ratio) of the copolymer of MPC and BMA is 0.05:99.95 to 67:33, and preferably 0.5:99.5 to 30:70 in particular.

The reason is that the moisture content of the polymer is varied in accordance with varying the composition ratio of MFC and BMA. In other words, the moisture content increases when the ratio of MPC increases, and the moisture content decrease when the ratio of BMA increases. Since the release rate of materials from a polymer membrane is varied depending on the moisture content of the polymer, the release rate of the encapsulated material can be controlled by varying the composition ratio. Endothelin Receptor Antagonist

The endothelin receptor antagonist used in the present invention can use 4,5-dihydro-[1H]-benz[g]indazol-3-carboxylic acid derivative represented by the above-mentioned formula (1) or salt thereof. For example, 4,5-dihydro-[1H]-benz[g]indazol-3-carboxylic acid derivative or salt thereof can be synthesized by way of the method described in Unexamined Japanese Patent No. H11-29569. Particularly, 3-carboxy-4,5-dihydro-1-[1-(3-ethoxyphenyl)propoxy]-7-(5-pyrimidinyl)methoxy-[1H]-benz[g]indazol represented by the above-mentioned formula (2) is effective in small amount so that it is preferably used.

As the endothelin receptor antagonist, (1S,2R,3S)-3-[2-(2-hydroxyethoxy)-4-methoxyphenyl]-1-[3,4-(methylenedioxy)phenyl]-5-propoxy-2-indane carboxylic acid represented by the above-mentioned structural formula (3) or [5S-[5α,6β,7α(R*)]]-2-butyl-5-(1,3-benzodioxy-ol-5-yl)-7-[(2-carboxypropyl)-4-methoxyphenyl]-6-dihydro-5H-cyclopenta[b]pyridine-6-carbocylic acid represented by the above-mentioned structural formula (4) can be used. Any one of compounds represented by the structural formulas (3) and (4) is effective in small amount.

(1S,2R,3S)-3-[2-(2-hydroxyethoxy)-4-methoxyphenyl]-1-[3,4-(methylenedioxy)phenyl]-5-propoxy-2-indane carboxylic acid (generic name: enrasentan) available from SmithKline Beecham can be used. Alternatively, the compound can be synthesized by way of the method described in 211th American Chemical Society (ACS), New Orleans: MEDI108, Mar1996.

[5S-[5α,6β,7α(R*)]]-2-butyl-5-(1,3-benzodioxy-ol-5-yl)-7-[(2-carboxypropyl)-4-methoxyphenyl]-6-dihydro-5H-cyclopenta[b]pyridine-6-carboxylic acid (cord No.: J-104132) available from Banyu Pharmaceutical (Merck)) can be used. Alternatively, the compound can be synthesized by way of any one of the methods described in Lynch JJ Jr and shen YT. Method of treating heart failure and ventricular dysfunction with endothelin antagonist and International Publication No. WO97/037665.

The composition for drug-eluting stent of the present invention can contain additives such as excipient, a binder, a stabilizer, a preservative, and a colorant generally used for a pharmaceutical formulation in addition to the above-mentioned component. Composition Ratio

The composition ratio (mass ratio) of poly (MPC-co-BMA) and the endothelin receptor antagonist in the composition for drug-eluting stent according to the present invention is preferably 0.01 to 49.99 parts, more preferably 0.1 to 49.9 parts of the endothelin receptor antagonist based on 100 parts of poly (MPC-co-BMA). Stent

The stent is not limited to the material, the shape, the size, or the like in particular as long as it can be placed at a lesioned site developed in an in vivo lumen such as a blood vessel. As means for placing at the lesioned site, a balloon expansion means can be used, and a self-expansion means using elasticity can be used when the stent is elastomer.

The material forming the stent can be selected appropriately depending on the site to be applied. For example, metallic materials, polymer materials, ceramics, carbon fibers, and the like can be included. When the stent is formed of metallic substance, metallic materials have superior intensity so that the stent can be certainly placed at the lesioned site. Alternatively, when the stent is formed of a polymer material, the polymer material has superior flexibility, whereby the stent has superior accessibility to the lesioned site.

The metallic material for the stent can include, for example, stainless steel, Ni-Ti alloy, tantalum, titanium, gold, platinum, inconel, iridium, tungsten, cobalt-based alloy, and the like.

The polymer material can include, for example, a biocompatible polymer material such as polytetrafluoroethylene, polyethylene, polypropylene, polyethylene terephthalate, cellulose acetate, cellulose nitrate and the like; polylactic acid; polyglycolic acid; a copolymer of lactic acid and glycolic acid; and biodegradable polymer material such as polycaprolactone, polyhydroxy and the like. Coating Method

The method for coating the composition for drug-eluting stent to the surface thereof is not limited in particular. However, the method can include a method in which the composition is melted and then coated to the surface of the stent; a method in which the stent is immersed in the composition to be coated; a method in which a solution is prepared by dissolving the composition in a solvent, the stent is immersed in this solution and pulled out, and then the solvent is removed by evaporation or by other manners; a method in which the composition is sprayed on the stent by using a spray, and then the solvent is removed by evaporation or by other manners.

The coating can be formed from a plurality of coating layers by coating more than two layers of the composition. In this case, the elution of the drag can be controlled depending on each layer by varying the composition ratio of MPC and BMA as mentioned above. For example, the concentration of BMA in the outer layer is decreased, and the concentration of BMA of the inner layer is increased so that the drug of the outer layer is eluted at an early stage, and the drug of the inner layer is eluted after long period is elapsed. Accordingly, the effect can be prolonged over a long term after stent placement.

The thickness of the coated composition is preferably not more than 1 µm. When thickness of the coated composition is not less than 1 µm, the composition exfoliates at the time of stent expansion, resulting in causing embolus.

### EXAMPLE

The present invention is described in detail using Examples as follows.

### Example 1

Poly (MPC-co-BMA) was prepared so that 10% by mass of MPC and 90% by mass of BMA were contained therein. Poly (MPC-co-BMA) was mixed with 0.3% of the compound represented by the structural formula (2), whereby the composition defined as Example 1 was prepared.

### Example 2

Poly (MPC-co-BMA) was prepared in a way similar to the method in Example 1, except that the compound was prepared so that 20% by mass of MPC and 80% by mass of BMA were contained therein.

### Example 3

Poly (MPC-co-BMA) was prepared in a way similar to the method in Example 1, except that the compound was prepared so that 30% by mass of MPC and 70% by mass of BMA were contained therein.

### Comparative Example 1

Poly (MPC-co-BMA) was prepared so that 10% by mass of MPC and 90% by mass of BMA were contained therein. The compound was defined as Comparative Example 1.

### Comparative Example 2

Poly (MPC-co-BMA) was prepared in a way similar to the method in Comparative Example 1, except that the compound was prepared so that 20% by mass of and 80% by mass of BMA were contained therein.

### Comparative Example 3

Poly (MPC-co-BMA) was prepared in a way similar to the method in Comparative Example 1, except that the compound was prepared so that 30% by mass of MPC and 70% by mass of BMA were contained therein.

### Test 1: Verification Test of Stenosis Development

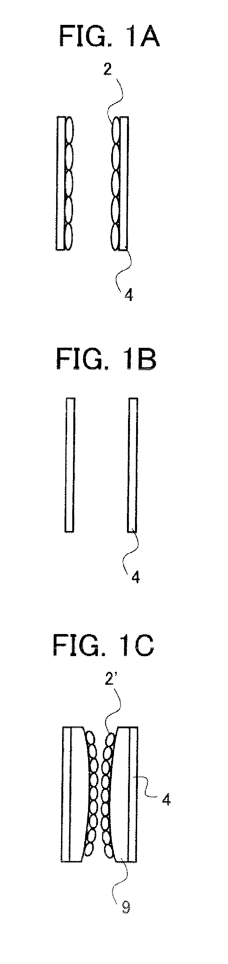

Endarterectomy was conducted in a rabbit common carotid artery, and then stenosis development was verified. Fig. 1 shows the pattern diagrams of the test. Fig. 1A shows the rabbit normal common carotid artery. The most inner side of tunica media of smooth muscle layer 4 is covered with one layer of endothelial cells 2. As shown in Fig. 1B, these endothelial cells 2 were removed by using a balloon catheter for removing embolus. As shown in Fig. 1C, smooth muscle cells are abnormally proliferated, thereby inducing stenosis due to intimal thickening 9 after six weeks since blood reflow. In addition, the inner side of the intimal thickening 9 is coated with regenerated endothelial cells 2' . The state of the common carotid artery after six weeks since blood reflow was verified with a microscope.

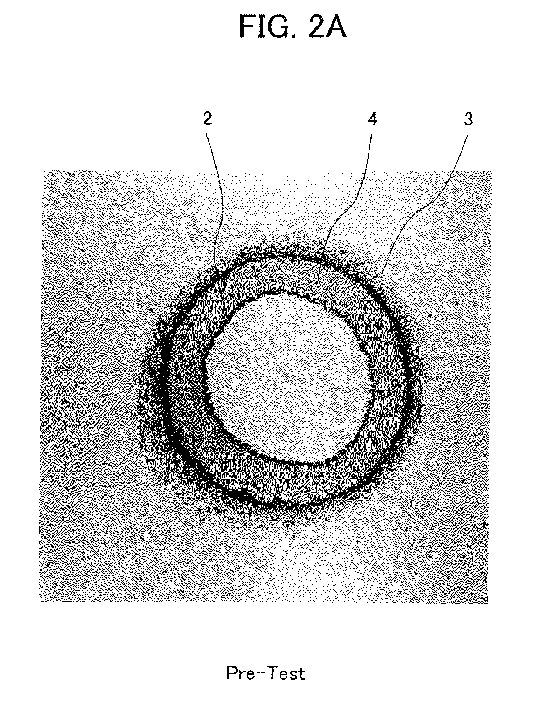

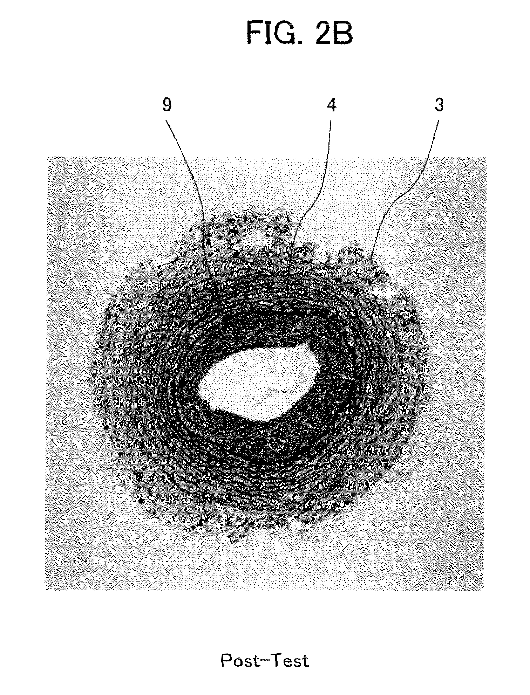

Fig. 2A shows an image of the common carotid artery tissue before endothelial cells are removed. Fig. 2B shows an image of stenosis due to intimal thickening after six weeks since removal of endothelial cells. As shown in Fig. 2A, a common carotid artery consists of adventitia 3 and tunica media of smooth muscle layer 4, and the surface of the vascular lumen is covered with endothelial cells 2. On the other hand, as is clear from Fig. 2B, pronounced intimal thickening 9 was induced after six weeks since removal of endothelial cells, and the vascular lumen was constricted. Test 2: Elution Test of Compound Represented by Structural Formula (2)

The elution test of the compound represented by the structural formula (2) from poly (MPC-co-BMA) was conducted. The test sample was prepared by coating sequentially the compositions from Examples 1 to 3 to a thin plate of stainless steel. The prepared sample was immersed in distilled water, and then the amount of the eluted compound represented by the structural formula (2) was measured with time.

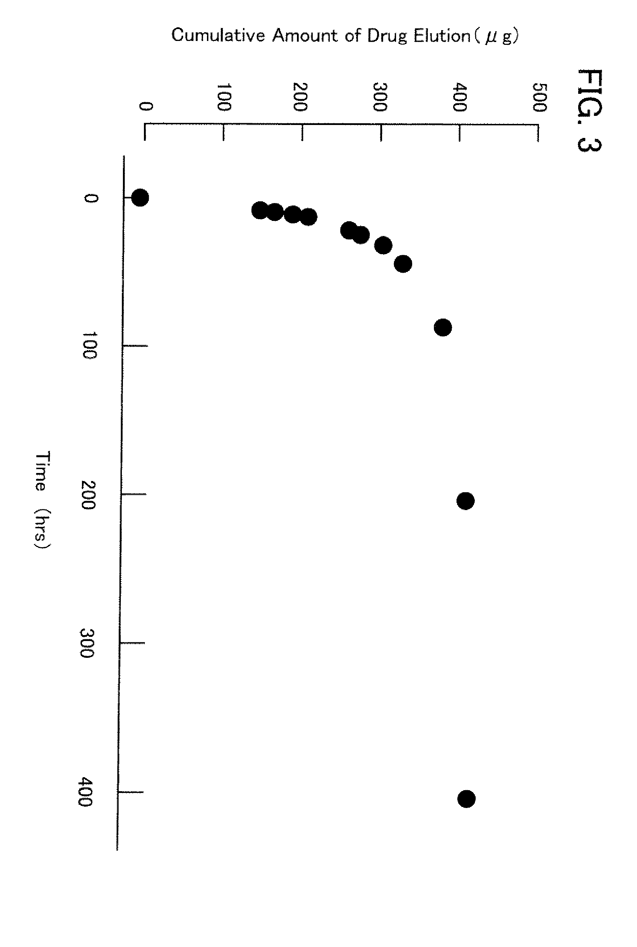

Fig. 3 shows a correlation with the time length after post-immersing and the cumulative amount of the compound represented by the structural formula (2). It can be verified that the compound represented by the structural formula (2) is eluted for about 100 hours. In addition, even after 100 hours, the compound represented by the structural formula (2) is eluted gradually for 400 hours, and thus it can be verified that controlled release is intended. Test 3: Stenosis Verification Test after Stent Insertion

The test samples were prepared by way of the methods in the following Preparations.

### Preparation 1

Drug-eluting stem was prepared by coating the surface of the stent with the structure shown in the following Table 1. Cordis and vascular stents (PQ294BLX) manufactured by Johnson & Johnson Corporation were used as the stent. First, the stent was immersed in the composition of Example 1 for about 1 minute, whereby the first layer of coating was provided thereon. After drying the stent, the stent was immersed in the composition of Example 2 for about 1 minute, whereby the second layer of coating was provided thereon. Finally, the stent was immersed in the composition of Example 3 for about 1 minute, whereby the third layer of coating was provided thereon. The stent prepared according to Preparation 1 was defined as stent 5.

**Table 1**

| | | MPC(%)-co-BMA(%) | | Final Concentration (%) | Concentration of Structural Formula (%) (2) |
|---|---|---|---|---|---|
| | | MPC(%) | BMA(%) | | |
| Inner Layer | Example 1 | 10 | 90 | 3 | 0.3 |
| Inter Layer | Example 2 | 20 | 80 | 3 | 0.3 |
| Outer Layer | Example 3 | 30 | 70 | 3 | 0.3 |

### Preparation 2

The compositions of Comparative Examples 1, 2, and 3 were coated sequentially on a cordis and vascular stent (PQ294BLX) manufactured by Johnson & Johnson Corporation in the same way as the method of Preparation 1. The stent prepared according to Preparation 2 was defined as stent 6.

### Preparation 3

The same stent as those used in Preparations 1 and 2 without any coating was defined as stent 7.

### Test

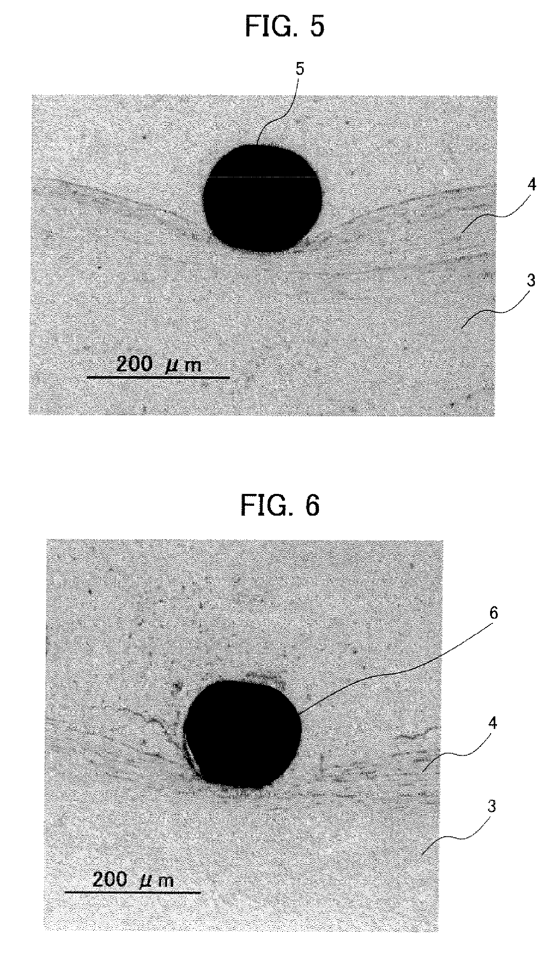

The stents were placed in rabbit common carotid artery immediately after endarterectomy was conducted by using the stents from Preparations 1 to 3. The common carotid artery in which the stent was placed after six weeks later was extracted and then searched histologically. Figs. 5 to 7 show the results.

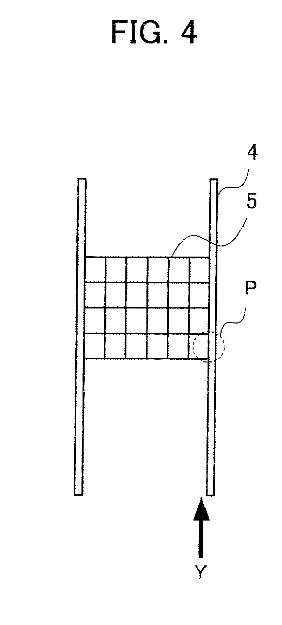

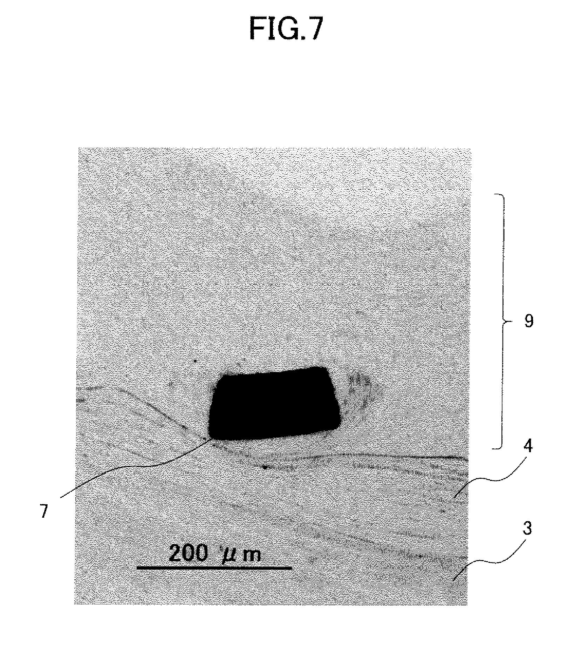

Fig. 4 shows a pattern diagrams illustrating that the drug-eluting stent 5 from Preparation 1 is placed in a blood vessel. Fig. 5 shows a tissue image of part P in view from Y-direction shown in Fig. 4, which illustrates a state around the metal stent after the placement of the drug-eluting stent 5 from Preparation 1 after six weeks. Figs. 6 and 7 show a tissue image of the same part as that shown in Fig. 5. As shown in Fig. 5, proliferative change was hardly observed around the circular metal of the stent 5 containing the compound represented by the structural formula (2) from Preparation 1. In addition, no stenosis of the vascular lumen was observed at all. Tunica media of smooth muscle layer 4 exists in contact with the lower hem of the stent 5 in Fig. 5. Adventitia 3 exists under tunica media of smooth muscle layer 4.

Fig. 6 shows a tissue image around the metal stent after six weeks since placement of the stent 6 from Preparation 2. When the compound represented by the structural formula (2) is not contained in the coating, the large amount of cell aggregation is observed around the metal stent, and a mild proliferative change began. Extremely mild stenosis of vascular lumen was observed.

Fig. 7 shows a tissue image around the metal stent after six weeks since placement of the stent 7 from Preparation 3. Severe stenosis of vascular lumen due to intimal thickening 9 was observed. It was verified that proliferated tissues completely covers around the trapezoid metal of the stent 7.

## Claims

1. A method for control of drug elution rate of an endothelin receptor antagonist from a coating composition for a drug-eluting stent comprising:
varying a composition ratio of a copolymer of 2-methacryloyloxyethyl phosphorylcholine and n-butyl methacrylate,
wherein the coating composition for a drug-eluting stent comprises the endothelin receptor antagonist and the copolymer of 2-methacryloyloxyethyl phosphorylcholine and n-butyl methacrylate.

2. The method for control of drug elution rate according to claim 1,
wherein the composition ratio by mass of 2-methacryloyloxyethyl phosphorylcholine and n-butyl methacrylate in the copolymer is 0.05:99.95 to 67:33.

3. The method for control of drug elution rate according to claim 1 or 2,
wherein the composition ratio by mass of 2-methacryloyloxyethyl phosphorylcholine and n-butyl methacrylate in the copolymer is 0.5:99.95 to 30:70.

4. The method for control of drug elution rate according to any one of claims 1 to 3, wherein the endothelin receptor antagonist is 4,5-dihydro-[1H]-benz[g]indazol-3-carboxylic acid derivative represented by the following structural formula (1) or salt thereof, 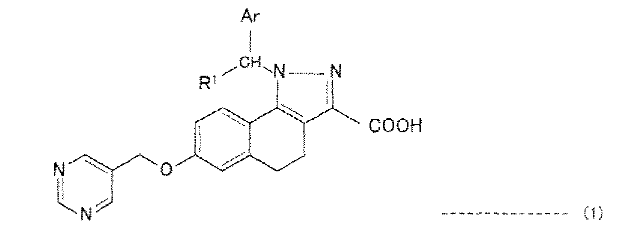 wherein Ar presents a substituted or unsubstituted aryl group; and R¹ represents a hydrogen atom, a substituted or unsubstituted lower alkyl group, a substituted or unsubstituted aryl group, a substituted or unsubstituted cycloalkyl group, or a substituted or unsubstituted heterocyclic group.

5. The method for control of drug elution rate according to claim 4,
wherein 4,5-dihydro-[1H]-benz[g]indazol-3-carboxylic acid derivative or salt thereof is 3-carboxy-4,5-dihydro-1-[1-(3-ethoxyphenyl)propoxy]-7-(5-pyrimidinyl)methoxy-[1H]-benz[g]indazol represented by the following structural formula (2).

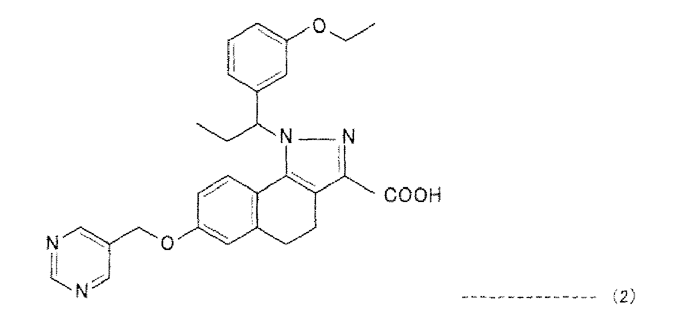

6. The method for control of drug elution rate according to any one of claims 1 to 3,
wherein the endothelin receptor antagonist is (1S,2R,3S)-3-[2-(2-hydroxyethoxy)-4-methoxyphenyl]-1-[3,4-(methylenedioxy)phenyl]-5-propoxy-2-indane carboxylic acid represented by the following structural formula (3) or [5S-[5α,6β,7α(R*)]]-2-butyl-5-(1,3-benzodioxy-ol-5-yl)-7-[(2-carboxypropyl)-4-methoxyphenyl]-6-dihydro-5H-cyclopenta[b]pyridine-6-carboxylic acid represented by the following structural formula (4).

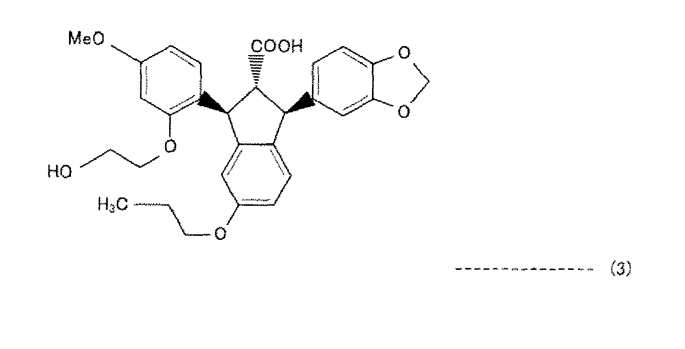

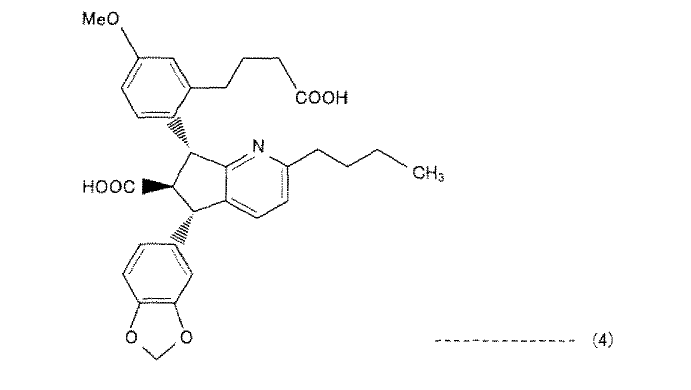

7. A composition for coating a drug-eluting stent comprising:
an endothelin receptor antagonist and a copolymer of 2-methacryloyloxyethyl phosphorylcholine and n-butyl methacrylate.

8. The composition for coating a drug-eluting stent according to claim 7, the composition ratio by mass of 2-methacryloyloxyethyl phosphorylcholine and n-butyl methacrylate in the copolymer is 0.05:99.95 to 67:33.

9. The composition for coating a drug-eluting stent according to claim 7 or 8,
wherein the composition ratio by mass of 2-methacryloyloxyethyl phosphorylcholine and n-butyl methacrylate in the copolymer is 0.5:99.95 to 30:70.

10. The composition for coating a drug-eluting stent according to any one of claims 7 to 9,
wherein the endothelin receptor antagonist is 4,5-dihydro-[1H]-benz[g]indazol-3-carboxylic acid derivative represented by the following structural formula (1) or salt thereof, 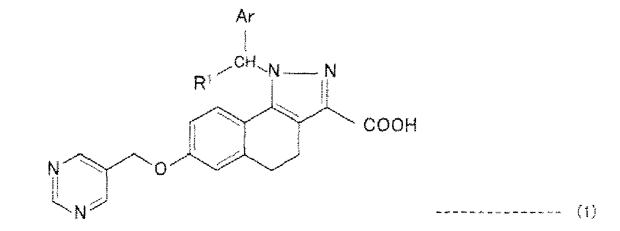 wherein Ar presents a substituted or unsubstituted aryl group; and R¹ represents a hydrogen atom, a substituted or unsubstituted lower alkyl group, a substituted or unsubstituted aryl group, a substituted or unsubstituted cycloalkyl group, or a substituted or unsubstituted heterocyclic group.

11. The composition for coating a drug-eluting stent according to claim 10,
wherein 4,5-dihydro-[1H]-benz[g]indazol-3-carboxylic acid derivative or salt thereof is 3-carboxy-4,5-dihydro-1-[1-(3-ethoxyphenyl)propoxy]-7-(5-pyrimidinyl)methoxy-[1H]-benz[g]indazol represented by the following structural formula (2).

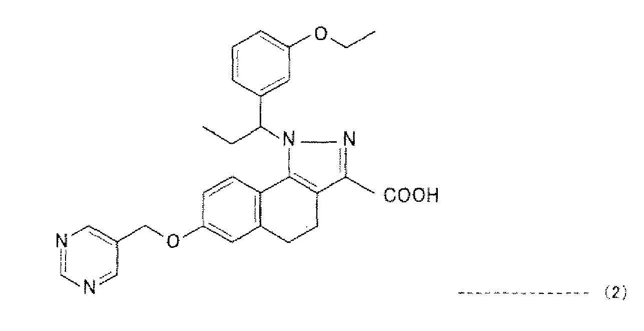

12. The composition for coating a drug-eluting stent according to any one of claims 7 to 9,
wherein the endothelin receptor antagonist is (1S,2R,3S)-3-[2-(2-hydroxyethoxy)-4-methoxyphenyl]-1-[3,4-(methylenedioxy)phenyl]-5-propoxy-2-indane carboxylic acid represented by the following structural formula (3) or [5S-[5α,6β,7α(R*)]]-2-butyl-5-(1,3-benzodioxy-ol-5-yl)-7-[{2-carboxypropyl)-4-methoxyphenyl]-6-dihydro-5H-cyclopenta[b]pyridine-6-carboxylic acid represented by the following structural formula (4).

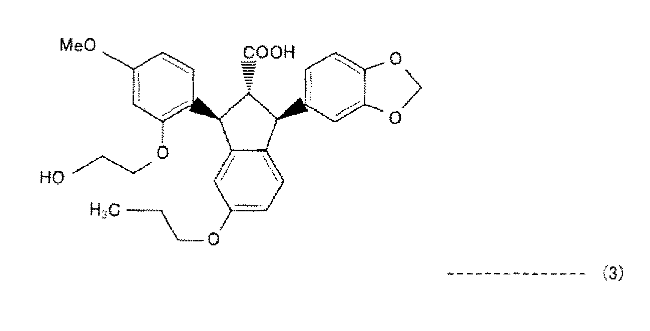

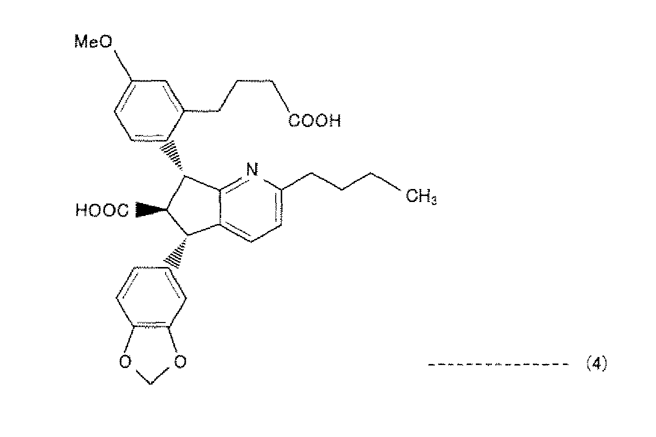

13. A drug-eluting stent formed by coating the composition for coating a drug-eluting stent according to any one of claims 7 to 12 thereon.

14. The drug-eluting stent according to claim 13,
wherein the coating comprises a plurality of coating layers, and the ratio of n-butyl methacrylate in the copolymer of 2-methacryloyloxyethyl phosphorylcholine and n-butyl methacrylate decreases sequentially from the inner layer to the outer layer.
